# EUROPEAN PATENT SPECIFICATION

(11) **EP 1 353 624 B1**
(45) Date of publication and mention of the grant of the patent: **18.03.2009**
(21) Application number: 01991084.3
(22) Date of filing: 12.12.2001
(51) Int. Cl.: A61K 6/00, A61K 31/445, A61K 47/02, A61K 9/16

(54) **PHARMACEUTICAL COMPOSITIONS OF A NON-ENTERIC COATED PROTON PUMP INHIBITOR WITH A CARBONATE SALT AND BICARBONATE SALT COMBINATION**
PHARMAZEUTISCHE ZUSAMMENSETZUNGEN EINES NICHTMAGENSAFTRESISTENTEN PROTONENPUMPEN-HEMMERS MIT EINER CARBONAT- UND BICARBONATSALZKOMBINATION
COMPOSITIONS PHARMACEUTIQUES D'UN INHIBITEUR DE LA POMPE A PROTONS NON GASTRO-RESISTANT AVEC UNE COMBINAISON DE SEL DE CARBONATE ET DE SEL DE BICARBONATE

(30) Priority: 28.12.2000 US 750430
(43) Date of publication of application: 22.10.2003
(73) Proprietor: TAP Pharmaceutical Products, Inc., Lake Forest, Illinois 60045 (US)
(72) Inventor: TANEJA, Rajneesh, Gurnee, IL 60031 (US); GUPTA, Pramod, Gurnee, IL 60031 (US)
(74) Representative: Modiano, Micaela Nadia
(86) International application number: PCT/US2001/048320
(87) International publication number: WO 2002/053097

(56) References cited:
- WO-A-01/03707
- US-A- 5 750 104
- US-A- 5 840 737
- SHARMA V K ET AL: "Oral pharmacokinetics of omeprazole and lansoprazole after single and repeated doses as intact capsules or as suspensions in sodium bicarbonate" ALIMENTARY PHARMACOLOGY AND THERAPEUTICS, vol. 14, no. 7, July 2000 (2000-07), pages 887-892, XP001181327 ISSN: 0269-2813
- SHARMA V K ET AL: "SIMPLIFIED LANSOPRAZOLE SUSPENSION-A LIQUID FORMULATION OF LANSOPRAZOLE EFFECTIVELY SUPPRESSES INTRAGASTRIC ACIDITY WHEN ADMINISTERED THROUGH A GASTROSTOMY" AMERICAN JOURNAL OF GASTROENTEROLOGY, NEW YORK, NY, US, vol. 94, no. 7, August 1999 (1999-08), pages 1813-1817, XP002907013 ISSN: 0002-9270
- SHARMA V K: "Comparison of 24-hour intragastric pH using four liquid formulations of lansoprazole and omeprazole." AMERICAN JOURNAL OF HEALTH-SYSTEM PHARMACY : AJHP : OFFICIAL JOURNAL OF THE AMERICAN SOCIETY OF HEALTH-SYSTEM PHARMACISTS. 1 DEC 1999, vol. 56, no. 23 Suppl 4, 1 December 1999 (1999-12-01), pages S18-S21, XP009030627 ISSN: 1079-2082
- DATABASE WPI Section Ch, Week 199335 Derwent Publications Ltd., London, GB; Class B02, AN 1993-278196 XP002280819 & JP 05 194225 A (YOSHITOMI PHARM IND KK) 3 August 1993 (1993-08-03)
- DATABASE WPIDS [Online] (COLUMBUS, OH, USA) COOK: 'Composition used for treating gastric and duodenal ulcers contains benzimidazole compound and e.g. sodium hydroxide', XP002951122 Retrieved from STN Database accession no. 2001-320522 & JP 2000 355540 A 26 December 2000

## Description

### Field of the Invention

The invention is directed to a use for manufacturing a medicament for treating gastric acid disorders, by administration to a patient in need of such treatment, a therapeutically effective amount of at least one non-enteric coated proton pump inhibitor in a pharmaceutically acceptable carrier; wherein said pharmaceutically acceptable carrier comprises a bicarbonate salt of a Group IA metal and a carbonate salt of a Group IA metal in an equimolar ratio; and a pharmaceutical composition of a non-enteric coated proton pump inhibitor in a pharmaceutically acceptable carrier including a bicarbonate salt of a Group IA metal and a carbonate salt of a Group IA metal in equimolar ratio. A presently preferred proton pump inhibitor is lansoprazole, a presently preferred bicarbonate salt is sodium bicarbonate, and a presently preferred carbonate salt is sodium carbonate. The composition is a fast-acting formulation which reduces the undesirable belching associated with proton pump inhibitor formulations that contain high doses of sodium bicarbonate.

### Background of the Invention

Lansoprazole is a substituted benzimidazole which inhibits gastric acid secretions. It belongs to a class of compounds called proton pump inhibitors (PPI). The key action mechanism of the PPIs is inhibition of H⁺/K⁺-adenosine triphosphate (also known as acid pump or proton pump), an enzyme present in the gastric parietal cells. These drugs are metabolized in the parietal cells to active sulfenamide metabolites that inactivate the sulfhydryl group of the proton pump, thus reducing the hydrogen ion secretion. Lansoprazole is a lipophilic weak base with poor aqueous solubility at low pH. It is unstable in low pH solutions and undergoes rapid acid-catalyzed degradation, though it is relatively stable at neutral or high pH.

Due to the pH sensitivity of lansoprazole described above, effective drug delivery is problematic, as the pH of the gastric environment is acidic and the pH of the intestinal region is relatively alkaline. For the drug to be therapeutically active after oral administration, it should be protected from the acid present in the gastric juices. Further, the drug should reach the upper small intestinal region in an intact, absorbable form, where the drug can be rapidly absorbed to reduce acid production.

Enteric coating is by far the most popular method of protecting an acid-labile drug from gastric degradation. In this method, either the drug particles or the dosage form is coated with a polymer that does not dissolve in the low pH gastric environment, but dissolves in the alkaline environment of the small intestine. Currently, lansoprazole is administered as enteric-coated granules filled in a hard gelatin capsule in solid dosage form (B. Delhotal Landes et al. in "Clinical Pharmacokinetics of Lansoprazole", Clin. Pharmacokinet., 28 (6) 1995). This enteric coat dissolves at a pH > 6.

Tableted effervescent dosage forms of enteric-coated proton pump inhibitors including sodium carbonate and bicarbonate are disclosed in WO 97/25030 and U.S. Patent No. 6,132,770. In addition, U.S. Patent No. 5,840,737 discloses a pharmaceutical composition including an aqueous solution/suspension of omeprazole or other substituted benzimidazoles in a carrier including a bicarbonate salt of a Group IA metal.

However, there are some problems associated with enteric-coated preparations. These preparations are difficult to formulate as liquids, which may inconvenience pediatric patients or a patient population which has difficulty in swallowing. Moreover, the enteric coating must dissolve before the drug may be available for absorption. Since dissolution of the enteric coating is pH-dependent, and the pH profile of the gastrointestinal tract in an individual is variable at different times and is dependent on numerous physiological factors (e.g., the fed or fasted state), variable dissolution times for the enteric coat and variable pharmacokinetic profiles in individuals may result.

The acid-labile drugs for oral administration may also be protected from gastric acidity by neutralizing the pH of the gastric fluid. Conventional liquid formulations incorporate an acid neutralizer and enteric-coated PPI to create a stable formulation such as WO 94/02140, which discloses a core composed of an antacid combination and United States Patent No. 6,096,340 which discloses an enteric-coated formulation containing omeprazole, a surface-active agent, a filler, a pharmaceutically acceptable alkaline agent and a binder.

Co-administration of enteric-coated omeprazole, another proton pump inhibitor drug (benzimidazole compound), with 8.4% sodium bicarbonate solution/suspension via the nasogastric tube, has been disclosed by Phillips et al. in "A Prospective Study of Simplified Omeprazole Suspension for the Prophylaxis of Stress-Related Mucosal Damage", Crit. Care Med, 1996, Vol. 24, No. 11, and Sharma et al. in "The Effects on Intragastric Acidity of Per-Gastronomy Administration of an Alkaline Suspension of Omeprazole", Aliment Pharmacol. Ther., 13:1091-1095 (1999). Before administering, the enteric-coated drug granules were shaken with the sodium bicarbonate solution for a sufficient time period until a milky white suspension resulted, to dissolve the enteric coating in the sodium bicarbonate solution. A large quantity of sodium bicarbonate must be administered with each dose of omeprazole, in the method described above. However, there is a major disadvantage in using large quantities of sodium bicarbonate orally, since sodium bicarbonate, upon neutralization in the gastric fluid, produces gases and results in belching (see e.g. U.S. Patent No. 5,840,737). This is detrimental to patients suffering from gastro-esophageal reflux disease.

In an attempt to reduce the amount of co-administered sodium bicarbonate, Phillips et al. (WO 00126185) found that only 10 milliliters of an 8.4 % sodium bicarbonate solution were sufficient to provide effective acid neutralization and protect the enteric-coated omeprazole from degradation in the gastric environment. However, there is still a need for a method of PPI administration which is even more effective. In particular, a method which avoids the difficulties associated with the enteric-coating, yet still provides sufficient stability for either solid or liquid formulations would be particularly advantageous.

### Brief Summary of the Invention

The invention is directed to a use for manufacturing a medicament for treating gastric acid disorders, by administration to a patient in need of such treatment, a therapeutically effective amount of at least one non-enteric coated proton pump inhibitor in a pharmaceutically acceptable carrier; wherein said pharmaceutically acceptable carrier comprises a bicarbonate salt of a Group IA metal and a carbonate salt of a Group IA metal in an equimolar ratio.

The invention is also directed to a pharmaceutical composition comprising: at least one non-enteric coated proton pump inhibitor in a pharmaceutically acceptable carrier; wherein said pharmaceutically acceptable carrier includes a bicarbonate salt of a Group IA metal and a carbonate salt of a Group IA metal in equimolar ratio.

In the method or the pharmaceutical compositions of the present invention, either the Group IA metal of the bicarbonate salt or the Group IA metal of the carbonate salt, or both may be sodium or potassium. The non-enteric coated proton pump inhibitor may be a substituted benzimidazole or pharmaceutically acceptable salt thereof; and the substituted benzimidazole may be lansoprazole or a pharmaceutically acceptable salt thereof. The molar ratio of the bicarbonate salt to the carbonate salt is one to one. A presently preferred bicarbonate salt is sodium bicarbonate, and a presently preferred carbonate salt is sodium carbonate. The pharmaceutically acceptable carrier may contain from about 125 mg to about 1000 mg of sodium bicarbonate; and from about 125 mg to about 1000 mg of sodium carbonate.

The invention is also directed to a non-enteric coated lansoprazole composition consisting essentially of a) lansoprazole without enteric coating; b) a bicarbonate salt of a Group IA metal; and c) a carbonate salt of a Group IA metal.

### Detailed Description of the Invention

The invention is directed to a use for manufacturing a medicament for treating gastric acid disorders, by administration to a patient in need of such treatment, a therapeutically effective amount of at least one non-enteric coated proton pump inhibitor in a pharmaceutically acceptable carrier; wherein said pharmaceutically acceptable carrier comprises a bicarbonate salt of a Group IA metal and a carbonate salt of a Group IA metal in an equimolar ratio; and a pharmaceutical composition of a non-enteric coated proton pump inhibitor in a pharmaceutically acceptable carrier including a bicarbonate salt of a Group IA metal and a carbonate salt of a Group IA metal in equimolar ratio. A presently preferred proton pump inhibitor is lansoprazole, a presently preferred bicarbonate salt is sodium bicarbonate, and a presently preferred carbonate salt is sodium carbonate. The composition is a fast-acting formulation which reduces the undesirable belching associated with proton pump inhibitor formulations that contain high doses of sodium bicarbonate. Detailed discussions of the compositions and methods follow.

### The Compositions

The compositions include a proton pump inhibitor and a combination of a carbonate salt and a bicarbonate salt in equimolar ratio. The metal cation can be the same for the carbonate salt as well as the bicarbonate salt, or the metal cation on the carbonate salt can be a different one from the metal cation on the bicarbonate salt. The most combination of carbonate salt and bicarbonate salt is an equimolar mixture of sodium carbonate and sodium bicarbonate, referred to as "carbicarb". Substitution of carbicarb for bicarbonate in proton pump inhibitor formulations has certain advantages: 1) carbicarb results in a reduction in carbon dioxide by-product upon neutralization by gastric acids; 2) due to the higher acid-neutralizing capacity of carbonate ions than bicarbonate ions, less of the combination is required for gastric acid neutralization. These advantages translate to more effective proton pump inhibitor formulations, as the decrease in carbon dioxide by-product means that undesirable belching subsequent to administration will be reduced, and since less neutralizing agent is required, a smaller pill may be formulated. Moreover, we have found that the amount of carbicarb to be utilized is dependent upon the conditions of the stomach, and is unrelated to the amount of proton pump inhibitor to be administered. In most compositions, between 250-2000 mg of carbicarb is sufficient with each dose of proton pump inhibitor.

The phrase "Group IA metal" as used herein describes lithium, potassium and sodium.

The phrase "bicarbonate salt" refers to a compound of the formula M⁺HCO₃⁻, wherein M⁺ is a Group IA metal as defined above. A presently preferred bicarbonate salt is sodium bicarbonate, NaHCO₃.

The phrase "carbonate salt" refers to a compound of the formula (M⁺)₂ CO₃⁻², wherein M⁺ is a Group IA metal as defined above. A presently preferred carbonate salt is sodium carbonate, Na₂CO₃.

Proton pump inhibitors include substituted benzimidazoles such as omeprazole, lansoprazole, pantoprazole, pariprazole and leminoprazole.

A presently preferred proton pump inhibitor is lansoprazole, shown below.

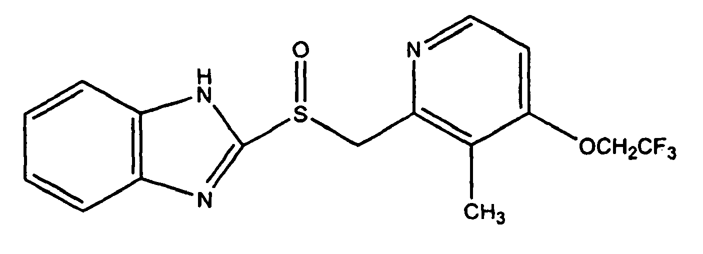

The proton pump inhibitors of the present invention can be used in the form of pharmaceutically acceptable salts derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge *et al.* describe pharmaceutically acceptable salts in detail in J. Pharmaceutical Sciences, 1977, 66: 1 et seq*.* The salts can be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl, chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

The proton pump inhibitors disclosed herein are not enteric-coated. By contrast, the commercially available proton pump inhibitors for oral administration are enteric-coated. The presently commercially available form of lansoprazole is PREVACID, a delayed release capsule available from TAP Pharmaceuticals, Inc. The delayed-release capsules contain enteric-coated lansoprazole, wherein the enteric coating is polymeric. Typical enteric coatings are made of cellulose acetate phthalate, hydroxypropylmethylcellulose phthalate, hydroxymethylcellulose acetate succinate, and methacrylic acid/acrylic acid copolymer (EUDRAGIT), among others.

The onset of action of a conventional proton pump inhibitor is about 1.5 to 2 hours. The presence of the enteric coating further delays this onset. Since the present compositions do not include enteric coatings, and do contain a fast-acting acid neutralizer, the length of time for onset of action is reduced. Therefore, the present compositions are advantageous in that they are fast-acting formulations.

As indicated above, the amount of the combination of the carbonate and bicarbonate salts does not depend upon the amount of the proton pump inhibitor utilized. However, the dosage range of the non-enteric coated proton pump inhibitor can range from approximately 0.5 mg/day to approximately 100 mg/day. The standard daily dosage is typically 10-60 mg non-enteric coated proton pump inhibitor, administered as a tablet, suspension or solution.

### The Uses

The pharmaceutical composition including the non-enteric coated proton pump inhibitor in a pharmaceutically acceptable carrier are useful in the treatment of various gastro-intestinal conditions.

The phrase "gastric acid disorders" as used herein includes active duodenal ulcers, gastric ulcers, gastro-esophageal reflux disease (GERD), severe erosive esophagitis, poorly responsive systematic GERD, and pathological hyper-secretory conditions such as Zollinger Ellison Syndrome, among others. Gastric acid disorders are those disorders caused by imbalances between acid and pepsin production, called aggressive factors, and mucus, bicarbonate, and prostaglandin production, called defensive factors.

The phrase "therapeutically effective amount" of the compound of the invention as used herein means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The non-enteric coated proton pump inhibitor is administered and dosed in accordance with good medical practice, taking into account the clinical condition of the individual patient, the site and method of administration, scheduling of administration, and other factors known to medical practitioners. The 'therapeutically effective amount" for purposes herein thus can readily be determines by such considerations as are known in the art. The amount must be effective to achieve improvement, including but not limited to, raising of gastric pH, reduced gastrointestinal bleeding, reduction in the need for blood transfusion, improved survival rate, more rapid recovery, or improvement or elimination of symptoms and other indicators as are selected as appropriate measures by those skilled in the art.

The phrase "pharmaceutically acceptable carrier" as used herein refers to a non-toxic compound such as lactose, starch, sucrose, glucose, methyl cellulose, magnesium stearate, dicalcium phosphate, calcium sulfate, mannitol or sorbitol among others.

The non-enteric coated proton pump inhibitor and combination of carbonate and bicarbonate salts can be administered in either solid or liquid dosage forms. A solid dosage form is illustrated in Example 4, and a liquid dosage form is illustrated in Example 5.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

In the uses of the present invention, the non-enteric coated proton pump inhibitor can be administered in various ways. The formulations can be made more palatable by adding flavorings such as chocolate, root beer, and others.

Additionally, the present invention can be manufactured by utilizing a micronized non-enteric coated proton pump inhibitor in place of the granules or powder in place of granules. Micronization is utilized in order to produce a particle having a smaller diameter. Micronization is the process by which solid drug particles are reduced in size. Since the dissolution rate is directly proportional to the surface area of the solid, and reducing the particle size increases the surface area, reducing the particle size increases the dissolution rate.

Although micronization results in increased surface area causing particle aggregation, which can negate the benefit of micronization and is an expensive manufacturing step, it does have the significant benefit of increasing the dissolution rate of relatively water insoluble drugs, such as omeprazole.

A pharmacological formulation of the non-enteric coated proton pump inhibitor utilized in the present invention can be administered orally to the patient.

These Examples are presented to describe preferred embodiments and utilities of the invention and are not meant to limit the invention unless otherwise stated in the claims appended hereto.

### Example 1

To illustrate the superiority of carbicarb solution over sodium bicarbonate solution, the comparative amounts of gas produced by each was studied. To quantify the gas produced by the neutralization of simulated gastric fluid (SGF) by carbicarb and by sodium bicarbonate *in vitro,* a standard CO₂ assay technique was utilized (see USP 24 -NF 19 Beta, The United States Pharmacopeia 2000, p. 306)

Aqueous solutions to be tested were made up from starting materials sodium carbonate, (anhydrous, available from Mallinckrodt) and sodium bicarbonate (available from Mallinckrodt). An 8.4% sodium bicarbonate solution and an 8.4% carbicarb solution (containing equimolar amounts of sodium bicarbonate and sodium carbonate) were tested as follows.

The specimen gas was generated by reacting either 450 mL simulated gastric fluid (SGF) with 50 mL of the carbicarb solution; or by reacting 45 ml of simulated gastric fluid with 5 ml of sodium bicarbonate solution in a 500 mL Erlenmeyer flask. The volume of reactants was reduced in the latter case, otherwise the volume of gas generated was too large to be determined in the Orsat Apparatus.

To make Simulated Gastric Fluid (SGF) 1 g sodium chloride was transferred to a 500-mL volumetric flask. After adding 1.6 g pepsin, 25 mL 2 N hydrochloric acid was added and swirled to mix. The resultant solution could then be diluted to the appropriate volume with water.

The amount of gas generated in a twenty-minute time period was measured in an Orsat Glass Vapor Absorption Apparatus, by the standard assay technique. Two tests were run for each solution. The volume of CO₂ generated was calculated from the difference in the initial volume of gas produced (ml) and the final volume of gas remaining (ml).

Table 1 demonstrates that, on average, there was a fourteen-fold reduction in the total gas generated and a two-fold reduction in the volume of CO₂ generated in the SGF-carbicarb reaction compared to the SGF-sodium bicarbonate reaction.

**Table 1**

| Volumes of total gas and carbon dioxide generated by the neutralization of simulated gastric fluid (SGF) with carbicarb and sodium bicarbonate | | | | |
|---|---|---|---|---|
| Test Solution | Replicate No. | Initial Gas Volume (mL) | Residual Gas Volume (mL) | Volume of CO₂ (mL) |
| 450 mL SGF and 50 mL 8.4% carbicarb solution | 1 | 60.0 | 39.0 | 21.0 |
| 450 mL SGF and 50 mL 8.4% carbicarb solution | 2 | 62.0 | 38.5 | 23.5 |
| 45 mL SGF and 5 mL 8.4% sodium bicarbonate solution | 1 | 93.0 | 90.1 | 2.9 |
| 45 mL SGF and 5 mL 8.4% sodium bicarbonate solution | 2 | 82.0 | 76.7 | 5.3 |
| 450 mL SGF and 50 mL 8.4% sodium bicarbonate solution | 1 | 930.0 | 901.0 | 29.0 |
| 450 mL SGF and 50 mL 8.4% sodium bicarbonate solution | 2 | 820.0 | 767.0 | 53.0 |

### Example 2

To determine and compare the gastric acid neutralizing capacity of sodium bicarbonate solution to carbicarb solution, the following experiment was performed.

Aqueous solutions to be tested were made up from starting materials sodium carbonate, (anhydrous, available from Mallinckrodt) and sodium bicarbonate (available from Mallinckrodt). An 8.4% sodium bicarbonate solution and an 8.4% carbicarb solution (containing equimolar amounts of sodium bicarbonate and sodium carbonate) were tested as follows.

50 mL simulated gastric fluid (SGF, prepared according to the procedure described in Example 1) was pipetted into an Erlenmeyer flask. Four drops of methyl red, then four drops of phenolphthalein were then added to the flask. The pH of solution was monitored with a pH electrode. The solution to be tested was added to a 5-mL buret. The gastric media in the flask was then titrated with the test solution to an endpoint within 0.2 pH units of pH 6.5 (as indicated by the color change and pH reading). Then, titration was resumed, to an endpoint within 0.2 pH units of pH 8.0 (as indicated by the color change and pH reading).

The endpoint was reached with the addition of 4.71 mL of sodium bicarbonate into the gastric media (Table 2). Similarly, the endpoint was reached with the addition of 3.32 mL of carbicarb solution into the gastric media (Table 2). Direct comparison of the two titrations (Table 2) shows that more of the sodium bicarbonate solution (at the same concentration) was required for the neutralization of the simulated gastric fluid. Thus, in the stomach, less carbicarb would be required to neutralize a given amount of gastric fluid.

**Table 2**

| Neutralization of the Simulated Gastric Fluid Acidity by Carbicarb and Sodium Bicarbonate Solutions | | |
|---|---|---|
| | Volume of Acid Neutralizer Solution | |
| | Required (ml) | |
| pH of simulated gastric fluid | 8.4% | 8.4% Sodium |
| after adding neutralizer | Carbicarb | Bicarbonate solution |
| | solution | |
| 1.2 | 0 | 0 |
| 2.0 | 2.57 | 3.47 |
| 3.0 | 3.05 | 4.09 |
| 4.0 | 3.13 | 4.19 |
| 5.0 | 3.19 | 4.27 |
| 6.0 | 3.26 | 4.48 |
| 7.0 | 3.40 | 5.00 |
| 8.0 | 3.53 | 6.20 |

### Example 3

Lansoprazole degrades in acid and is stable in base. This study was performed to determine 1) how quickly lansoprazole degrades in simulated gastric fluid (SGF); and 2) whether a high pH-buffering agent (carbicarb which is an equimolar mixture of sodium carbonate and sodium bicarbonate) could be used to retard the degradation of lansoprazole in SGF. All the experiments were conducted at room temperature (22°C ± 2°C).

The PPI test sample included lansoprazole (30 mg); mannitol (60 mg), meglumine (30 mg) and sodium hydroxide (3 mg).

First, 50.0 mL simulated gastric fluid (SGF, prepared according to the procedure described in Example 1), the PPI test sample and a stir bar were added to each of 6 separate 100-mL beakers labeled (consecutively) as 0, 5, 15, 30,45, and 60 minutes. Then 5.0 mL 2 N sodium hydroxide solution was added to the 0-minutes beaker with mixing. To each of the 5 remaining beakers, 10.0 mL of 8.4% carbicarb solution was added and stirred to mix. At the appropriate time interval (corresponding to the individual beaker numbers), 5.0 mL 2 N sodium hydroxide solution was added to stop the reaction. A portion of the resulting solution was diluted with pH 10 diluent. 10 µL of the resulting solution was then injected into the chromatographic system for assay.

The results are shown in Table 3. At 60 minutes, 98% of the drug that was reconstituted with water had degraded. In comparison, only 3.5 % of the drug that was constituted with carbicarb solution had degraded.

Lansoprazole, when administered with carbicarb into simulated gastric fluid (SGF), was stable for at least 60 minutes (< 5% degradation). Carbicarb neutralizes the acidity of simulated gastric fluid (SGF), thereby ensuring the stability and hence clinical utility of lansoprazole.

**Table 3**

| The stability of lansoprazole with and without carbicarb in simulated gastric fluid (SGF). | | |
|---|---|---|
| | % labeled amount of lansoprazole remaining | |
| Time in SGF (minutes) | Sample in water | Sample in carbicarb solution |
| 0 | 99.5 | 98.0 |
| 5 | 39.9 | 96.7 |
| 15 | 10.1 | 97.4 |
| 30 | 4.1 | 95.8 |
| 45 | 2.7 | 94.4 |
| 60 | 2.0 | 96.5 |

### Example 4

Granular formulations of lansoprazole, including carbicarb were also made and tested. The stability of the granular formulations was tested according to the procedure of Example 3. The granular formulations of lansoprazole for this example were prepared as follows.

60 gm sucrose (Superior coffee, Bensenville, IL) was dissolved in water (HPLC grade, Fisher Scientific, Pittsburgh, PA) with gentle heating to form a 60 % solution. Then 46.93 gm of sodium carbonate (Fisher Scientific, Pittsburgh, PA) and 37.17 gm of sodium bicarbonate (Fisher Scientific, Pittsburgh, PA) were mixed together thoroughly. Subsequently, 35 gm of this mixture (carbicarb), 7.5 gm lactose and 1.5 gm lansoprazole (Takeda Chemical Industries, Osaka, Japan) were transferred to a mortar and mixed vigorously.

6 ml of the 60 % sucrose solution was gradually added to the mortar while mixing with a pestle to form a coherent, wetted mass. This coherent mass was passed through a 10-mesh screen and the resulting granules were dried at 50°C for 12 hours.

Upon testing by the procedure described in Example 3, the results indicated that lansoprazole, when formulated with carbicarb as granules, was stable in simulated gastric fluid for at least 60 minutes.

### Example 5

The granular formulation formed according to the procedure of Example 4 was mixed with water to form a suspension for oral administration, as follows.

The coherent mass containing lansoprazole, carbicarb, lactose and sucrose solution was prepared as described above, in Example 3. This coherent mass was passed through a 20-mesh screen and the resulting granules were dried at 50°C for 12 hours. Granules containing 30-mg lansoprazole were transferred to an amber color container along with and an equal weight of flavor granules.
10 ml of water ((HPLC grade, Fisher Scientific, Pittsburgh, PA) was added to the container with gentle shaking to reconstitute the suspension. The resulting suspension of Lansoprazole was tested for stability in simulated gastric fluid as described earlier in Experiment 3.

As in the previous Example, lansoprazole suspension, when reconstituted from lansoprazole/carbicarb granules was stable in simulated gastric fluid for at least 60 minutes.

Additionally, the granules prepared in Examples 4 and 5 were kept at 22°C for 21 days in closed containers. They were then tested for potency and the presence of related substances; and lansoprazole in the formulations was found to be stable at the conclusion of the study.

## Claims

1. Use for manufacturing a medicament for treating gastric acid disorders, by administration to a patient in need of such treatment of a therapeutically effective amount, of at least one non-enteric coated proton pump inhibitor in a pharmaceutically acceptable carrier;
wherein said pharmaceutically acceptable carrier comprises a bicarbonate salt of a Group IA metal and a carbonate salt of a Group IA metal in an equimolar ratio.

2. The use of claim 1 wherein said Group IA metal of said bicarbonate salt is sodium.

3. The use of claim 1 wherein said Group IA metal of said carbonate salt is sodium.

4. The use of claim 1 wherein said Group IA metal of said bicarbonate salt is potassium.

5. The use of claim 1 wherein said Group IA metal of said carbonate salt is potassium.

6. The use of claim 1 wherein said non-enteric coated proton pump inhibitor is a substituted benzimidazole or pharmaceutically acceptable salt thereof

7. The use of claim 6 wherein said substituted benzimidazole is lansoprazole or a pharmaceutically acceptable salt thereof.

8. The use of claim 1 wherein said proton pump inhibitor is omeprazole, lansoprazole, pantoprazole, pariprazole or leminoprazole.

9. The use of claim 2 wherein said bicarbonate salt of said Group IA metal is sodium bicarbonate.

10. The use of claim 3 wherein said carbonate salt of said Group IA metal is sodium carbonate.

11. The use of claim 1 or 8 wherein said bicarbonate salt of a Group IA metal is sodium bicarbonate and said carbonate salt of a Group IA metal is sodium carbonate.

12. The use of claim 9 wherein said pharmaceutically acceptable carrier contains from about 125 mg to about 1000 mg of sodium bicarbonate.

13. The use of claim 10 or 12 wherein said pharmaceutically acceptable carrier contains from about 125 mg to about 1000 mg of sodium carbonate.

14. The use according to claims 10 and 11;
wherein said pharmaceutically acceptable carrier comprises an equimolar ratio of sodium carbonate to sodium bicarbonate.

15. The use of claim 14 wherein said pharmaceutically acceptable carrier contains from about 125 mg to about 1000 mg of sodium carbonate, and from about 125 mg to about 1000 mg of sodium bicarbonate.

16. A pharmaceutical composition comprising:
at least one non-enteric coated proton pump inhibitor in a pharmaceutically acceptable carrier;
wherein said pharmaceutically acceptable carrier comprises a bicarbonate salt of a Group IA metal and a carbonate salt of a Group IA metal in an equimolar ratio.

17. The composition of claim 16 wherein said Group IA metal of said bicarbonate salt is sodium.

18. The composition of claim 16 wherein said Group IA metal of said carbonate salt is sodium.

19. The composition of claim 16 wherein said Group IA metal of said bicarbonate salt is potassium.

20. The composition of claim 16 wherein said Group IA metal of said carbonate salt is potassium.

21. The composition of claim 16 wherein said bicarbonate salt of a Group IA metal is sodium bicarbonate and said carbonate salt of a Group IA metal is sodium carbonate.

22. The composition of claim 16 wherein said non-enteric coated proton pump inhibitor is a substituted benzimidazole or pharmaceutically acceptable salt thereof.

23. The composition of claim 22 wherein said benzimidazole is lansoprazole or a pharmaceutically acceptable salt thereof.

24. The composition of claim 16 or 21 wherein said proton pump inhibitor is omeprazole, lansoprazole, pantoprazole, pariprazole or leminoprazole.

25. The composition of claim 17 wherein said bicarbonate salt of said Group IA metal is sodium bicarbonate.

26. The composition of claim 18 wherein said carbonate salt of said Group IA metal is sodium carbonate.

27. The composition of claim 16 wherein said pharmaceutically acceptable carrier contains from about 125 mg to about 1000 mg of sodium carbonate and from about 125 mg to about 1000 mg of sodium bicarbonate.

28. A non-enteric coated lansoprazole composition consisting essentially of:
a) lansoprazole without enteric coating; and
b) a bicarbonate salt of a Group IA metal and a carbonate salt of a Group IA metal in an equimolar ratio.

29. The composition of claim 28 wherein said bicarbonate salt of said Group IA metal is sodium bicarbonate.

30. The composition of claim 28 wherein said carbonate salt of said Group IA metal is sodium carbonate.

31. The composition of claim 28 having from about 125 mg to about 1000 mg of sodium carbonate, and from about 125 mg to about 1000 mg of sodium bicarbonate.

## Patentansprüche

1. Verwendung zur Herstellung eines Medikaments zur Behandlung von Magensäurestörungen durch Verabreichung mindestens eines nicht magensaftresistenten Protonenpumpen-Hemmers in einem pharmazeutisch verträglichen Träger an einen Patienten, der eine solche Behandlung benötigt;
worin der pharmazeutisch verträgliche Träger ein Bicarbonatsalz eines Metalls der Gruppe IA und ein Carbonatsalz eines Metalls der Gruppe IA in einem äquimolaren Verhältnis umfasst.

2. Die Verwendung gemäß Anspruch 1, worin das Gruppe IA-Metall des Bicarbonatsalzes Natrium ist.

3. Die Verwendung gemäß Anspruch 1, worin das Gruppe IA-Metall des Carbonatsalzes Natrium ist.

4. Die Verwendung gemäß Anspruch 1, worin das Gruppe IA-Metall des Bicarbonatsalzes Kalium ist.

5. Die Verwendung gemäß Anspruch 1, worin das Gruppe IA-Metall des Carbonatsalzes Kalium ist.

6. Die Verwendung gemäß Anspruch 1, worin der nicht magensaftresistente Protonenpumpen-Hemmer ein substituiertes Benzimidazol oder ein pharmazeutisch verträgliches Salz davon ist.

7. Die Verwendung gemäß Anspruch 6, worin das substituierte Benzimidazol Lansoprazol oder ein pharmazeutisch verträgliches Salz davon ist.

8. Die Verwendung gemäß Anspruch 1, worin der Protonenpumpen-Hemmer Omeprazol, Lansoprazol, Pantoprazol, Pariprazol oder Leminoprazol ist.

9. Die Verwendung gemäß Anspruch 2, worin das Bicarbonatsalz des Gruppe IA-Metalls Natriumbicarbonat ist.

10. Die Verwendung gemäß Anspruch 3, worin das Carbonatsalz des Gruppe IA-Metalls Natriumcarbonat ist.

11. Die Verwendung gemäß Anspruch 1 oder 8, worin das Bicarbonatsalz eines Gruppe IA-Metalls Natriumbicarbonat ist und das Carbonatsalz eines Gruppe IA-Metalls Natriumcarbonat ist.

12. Die Verwendung gemäß Anspruch 9, worin der pharmazeutisch verträgliche Träger von ungefähr 125 mg bis ungefähr 1000 mg Natriumbicarbonat enthält.

13. Das Verwendung gemäß Anspruch 10 oder 12, worin der pharmazeutisch verträgliche Träger von ungefähr 125 mg bis ungefähr 1000 mg Natriumcarbonat enthält.

14. Die Verwendung gemäß den Ansprüchen 10 und 11, worin der pharmazeutisch verträgliche Träger ein äquimolares Verhältnis von Natriumcarbonat zu Natriumbicarbonat umfasst.

15. Die Verwendung gemäß Anspruch 14, worin der pharmazeutisch verträgliche Träger von ungefähr 125 mg bis ungefähr 1000 mg Natriumcarbonat und von ungefähr 125 mg bis ungefähr 1000 mg Natriumbicarbonat enthält.

16. Eine Pharmazeutische Zusammensetzung, die Folgendes umfasst :
mindestens einen nicht magensaftresistenten Protonenpumpen-Hemmer in einem pharmazeutisch verträglichen Träger;
worin der pharmazeutisch verträgliche Träger ein Bicarbonatsalz eines Metalls der Gruppe IA und ein Carbonatsalz eines Metalls der Gruppe IA in einem äquimolaren Verhältnis umfasst.

17. Die usammensetzung gemäß Anspruch 16, worin das Gruppe IA-Metall des Bicarbonatsalzes Natrium ist.

18. Die Zusammensetzung gemäß Anspruch 16, worin das Gruppe IA-Metall des Carbonatsalzes Natrium ist.

19. Die Zusammensetzung gemäß Anspruch 16, worin das Gruppe IA-Metall des Bicarbonatsalzes Kalium ist.

20. Die Zusammensetzung gemäß Anspruch 16, worin das Gruppe IA-Metall des Carbonatsalzes Kalium ist.

21. Die Zusammensetzung gemäß Anspruch 16, worin das Bicarbonatsalz eines Gruppe IA-Metalls Natriumbicarbonat ist und das Carbonatsalz eines Gruppe IA-Metalls Natriumcarbonat ist.

22. Die Zusammensetzung gemäß Anspruch 16, worin der nicht magensaftresistente Protonenpumpen-Hemmer ein substituiertes Benzimidazol oder ein pharmazeutisch verträgliches Salz davon ist.

23. Die Zusammensetzung gemäß Anspruch 22, worin das Benzimidazol Lansoprazol oder ein pharmazeutisch verträgliches Salz davon ist.

24. Die Zusammensetzung gemäß Anspruch 16 oder 21, worin der Protonenpumpen-Hemmer Omeprazol, Lansoprazol, Pantoprazol, Pariprazol oder Leminoprazol ist.

25. Die Zusammensetzung gemäß Anspruch 17, worin das Bicarbonatsalz des Gruppe IA-Metalls Natriumbicarbonat ist.

26. Die Zusammensetzung gemäß Anspruch 18, worin das Carbonatsalz des Gruppe IA-Metalls Natriumcarbonat ist.

27. Die Zusammensetzung gemäß Anspruch 16, worin der pharmazeutisch verträgliche Träger von ungefähr 125 mg bis ungefähr 1000 mg Natriumcarbonat und von ungefähr 125 mg bis ungefähr 1000 mg Natriumbicarbonat enthält.

28. Eine nicht magensaftresistente Lansoprazolzusammensetzung, im Wesentlichen bestehend aus:
a) Lansoprazol ohne magensaftresistente Beschichtung; und
b) einem Bicarbonatsalz eines Metalls der Gruppe IA und einem Carbonatsalz eines Metalls der Gruppe IA in einem äquimolaren Verhältnis.

29. Die Zusammensetzung gemäß Anspruch 28, worin das Bicarbonatsalz des Gruppe IA-Metalls Natriumbicarbonat ist.

30. Die Zusammensetzung gemäß Anspruch 28, worin das Carbonatsalz des Gruppe IA-Metalls Natriumcarbonat ist.

31. Die Zusammensetzung gemäß Anspruch 28, die von ungefähr 125 mg bis ungefähr 1000 mg Natriumcarbonat und von ungefähr 125 mg bis ungefähr 1000 mg Natriumbicarbonat hat.

## Revendications

1. Utilisation pour la préparation d'un médicament destiné au traitement des troubles de l'acidité gastrique, par administration à un malade ayant besoin d'un tel traitement d'une quantité thérapeutiquement efficace d'au moins un inhibiteur de la pompe à protons à enrobage non gastrorésistant dans un véhicule pharmaceutiquement acceptable ;
où ledit véhicule pharmaceutiquement acceptable comprend un sel bicarbonate d'un métal du groupe IA et un sel carbonate d'un métal d'un groupe IA dans un rapport équimolaire.

2. Utilisation selon la revendication 1, où ledit métal du groupe IA dudit sel bicarbonate est le sodium.

3. Utilisation selon la revendication 1, où ledit métal du groupe IA dudit sel carbonate est le sodium.

4. Utilisation selon la revendication 1, où ledit métal du groupe IA dudit sel bicarbonate est le potassium.

5. Utilisation selon la revendication 1, où ledit métal du groupe IA dudit sel carbonate est le potassium.

6. Utilisation selon la revendication 1, où ledit inihibiteur de la pompe à protons à enrobage non gastrorésistant est un benzimidazole substitué ou un sel pharmaceutiquement acceptable de celui-ci.

7. Utilisation selon la revendication 6, où ledit benzimidazole substitué est le lansoprazole ou un sel pharmaceutiquement acceptable de celui-ci.

8. Utilisation selon la revendication 1, où ledit inhibiteur de la pompe à protons est l'oméprazole, le lansoprazole, le pantoprazole, le pariprazole ou le léminoprazole.

9. Utilisation selon la revendication 2, où ledit sel bicarbonate dudit métal du groupe IA est le bicarbonate de sodium.

10. Utilisation selon la revendication 3, où ledit sel carbonate dudit métal du groupe IA est le carbonate de sodium.

11. Utilisation selon la revendication 1 ou 8, où ledit sel bicarbonate d'un métal du groupe IA est le bicarbonate de sodium et ledit sel carbonate d'un métal du groupe IA est le carbonate de sodium.

12. Utilisation selon la revendication 9, où ledit véhicule pharmaceutiquement acceptable contient d'environ 125 mg à environ 1 000 mg de bicarbonate de sodium.

13. Utilisation selon la revendication 10 ou 12, où ledit véhicule pharmaceutiquement acceptable contient d'environ 125 mg à environ 1 000 mg de carbonate de sodium.

14. Utilisation selon les revendications 10 et 11, où ledit véhicule pharmaceutiquement acceptable comprend un rapport équimolaire de carbonate de sodium et de bicarbonate de sodium.

15. Utilisation selon la revendication 14, où ledit véhicule pharmaceutiquement acceptable contient d'environ 125 mg à environ 1 000 mg de carbonate de sodium et d'environ 125 mg à environ 1 000 mg de bicarbonate de sodium.

16. Composition pharmaceutique comprenant :
au moins un inhibiteur de la pompe à protons à enrobage non gastrorésistant dans un véhicule pharmaceutiquement acceptable ;
où ledit véhicule pharmaceutiquement acceptable comprend un sel bicarbonate d'un métal du groupe IA et un sel carbonate d'un métal du groupe IA dans un rapport équimolaire.

17. Composition selon la revendication 16, où ledit métal du groupe IA dudit sel bicarbonate est le sodium.

18. Composition selon la revendication 16, où ledit métal du groupe IA dudit sel carbonate est le sodium.

19. Composition selon la revendication 16, où ledit métal du groupe IA dudit sel bicarbonate est le potassium.

20. Composition selon la revendication 16, où ledit métal du groupe IA dudit sel carbonate est le potassium.

21. Composition selon la revendication 16, où ledit sel bicarbonate d'un métal du groupe IA est le bicarbonate de sodium et ledit sel carbonate d'un métal du groupe IA est le carbonate de sodium.

22. Composition selon la revendication 16, où ledit inhibiteur de la pompe à protons à enrobage non gastrorésistant est un benzimidazole substitué ou un sel pharmaceutiquement acceptable de celui-ci.

23. Composition selon la revendication 22, où ledit benzimidazole est le lansoprazole ou un sel pharmaceutiquement acceptable de celui-ci.

24. Composition selon la revendication 16 ou 21, où ledit inhibiteur de la pompe à protons est l'oméprazole, le lansoprazole, le pantoprazole, le pariprazole ou le léminoprazole.

25. Composition selon la revendication 17, où ledit sel bicarbonate dudit métal du groupe IA est le bicarbonate de sodium.

26. Composition selon la revendication 18, où ledit sel carbonate dudit métal du groupe IA est le carbonate de sodium.

27. Composition selon la revendication 16, où ledit véhicule pharmaceutiquement acceptable contient d'environ 125 mg à environ 1 000 mg de carbonate de sodium et d'environ 125 mg à environ 1 000 mg de bicarbonate de sodium.

28. Composition de lansoprazole à enrobage non gastrorésistant consistant essentiellement en :
a) du lansoprazole sans enrobage gastrorésistant ; et
b) un sel bicarbonate d'un métal du groupe IA et un sel carbonate d'un métal du groupe IA dans un rapport équimolaire.

29. Composition selon la revendication 28, où ledit sel bicarbonate dudit métal du groupe IA est le bicarbonate de sodium.

30. Composition selon la revendication 28, où ledit sel carbonate dudit métal du groupe IA est le carbonate de sodium.

31. Composition selon la revendication 28, ayant d'environ 125 mg à environ 1 000 mg de carbonate de sodium et d'environ 125 mg à environ 1 000 mg de bicarbonate de sodium.
